# FASCICULE DE BREVET EUROPEEN

(11) **EP 0 539 363 B1**
(45) Date de publication et mention de la délivrance du brevet: **28.12.1994**
(21) Numéro de dépôt: 90914537.7
(22) Date de dépôt: 12.09.1990
(51) Int. Cl.: A61M 5/50

(54) **SERINGUE ANTI-CONTAGION**
ANTI-KONTAMINIERUNGSSPRITZE
ANTI-CONTAGION SYRINGE

(30) Priorité: 15.09.1989 FR 8912373
(43) Date de publication de la demande: 05.05.1993
(73) Titulaire: Denis, Jean Roger, F-87000 Limoges (FR)
(72) Inventeur: Denis, Jean Roger, F-87000 Limoges (FR)
(86) Numéro de dépôt international: FR9000656
(87) Numéro de publication internationale: WO9104066

(56) Documents cités:
- WO-A-89/00435
- FR-A- 2 298 340
- FR-A- 2 632 190
- US-A- 4 878 899
- US-A- 4 883 466

## Description

La présente invention concerne une seringue anti-contagion comprenant un corps cylindrique assemblé à une tête, un porte-aiguille pourvu d'un passage pour le liquide à injecter, monté coulissant dans la tête et maintenu dans celle-ci contre l'action d'un ressort, un piston cylindrique creux servant à la propulsion d'un liquide ou médicament jusqu'au passage ainsi qu'une aiguille montée sur le porte-aiguille.

Une telle seringue est décrite dans la demande de brevet WO-A-89/00345.

Avec la prolifération des maladies à virus, telles que : hépatite,sida,etc... l'aiguille infestée,après usage,présente un danger lors des manipulations des seringues.

Les réalisations présentées comportent certains défauts qui rendent les seringues impropres à l'utilisation en milieu médical.

Ainsi,il est totalement exclus qu'il puisse demeurer la moindre bulle d'air dans une seringue remplie de médicament en vue d'une injection,que cette dernière soit:intra-musculaire,sous-cutanée et,à plus forte raison intra-veineuse

En effet la bulle d'air introduite dans le raiseau veineux d'un être humain,entraîne à coup sûr à une mort par embolie.

Ceci étant très clair et parfaitement impératif,une seringue,pour avoir une chance de fabrication doit obligatoirement être prugeable de son air résiduel.Il est donc nécessaire que le trou correspondant à celui de l'aiguille se situe dans une partie haute de la seringue,lorsque celle-çi se trouve dans la position dirigée vers le ciel.

D'autre part,deuxième point crucial: le corps médical veut pouvoir choisir le type d'aiguille correspondant à ses besoins,adapter cette dernière à la seringue de son choix et même changer de type d'aiguille en cours d'injection (mélange avec une grosse aiguille,puis injection avec une aiguille fine) avec la même seringue.

Le problème que se propose de résoudre l'invention est de réaliser une seringue avec laquelle les risques de contagion après usage par l'aiguille,soit minimisés,entièrement purgeable de son air résiduel,permettant une interchangeabilité de l'aiguille en cours d'usage,et,présentant un blocage final anti-retour.

Ce problème est résolu par une seringue anti-contagion comportant:
a) un corps (1) cylindrique assemblé à une tête (2) dans lequel est prévu une feuillure (21).
b) un porte-aiguille (5) pourvu d'un passage pour le liquide à injecter,monté coulissant dans la tête (2) maintenu dans celle-çi contre l'action d'un ressort (8),
c) un piston (10) cylindrique creux servant à la propulsion d'un liquide ou médicament,jusqu'au passage du porte-aiguille (5),
d) une aiguille montée sur le porte-aiguille (5) caractérisée en ce que :
e) l'aiguille est montée amovible sur l'embout (7),
f) le porte-aiguille (5) est muni d'une partie conique inversée dans le prolongement de la tête,en liaison avec le passage pour le liquide,pour permettre la récupération de l'air résiduel,
g) le piston cylindrique comprend un fond conique (22) en regard de la tête (2),cône comportant à sa base un anneau de fragilité (16),et,se terminant par une lèvrejoint d'étanchéïté (18) qui,en fin de course du ditpiston vient se dilater dans la feuillure (21) créant ainsi un blocage anti-retour définitif,
h) le porte-aiguille (5) comporte une couronne dentée (19) en regard de l'anneau de fragilité (16),prévue pour la découpe de cet anneau (16),
i) Le maintient du porte-aiguille (5) dans la tête (2) est obtenu par un dispositif de blocage conçu pour pouvoir libérer le porte-aiguille par une poussée du piston (10)
j) le piston creux (10) et la tête sont dimensionnés de façon à pouvoir loger le porte-aiguille muni de son aiguille losqu'après libération du dispositif de blocage par le piston, le porte-aiguille est propulsé sous l'effet du ressort (8) vers l'intérieur du piston (10),après avoir rompu l'anneau de fragilité (16)
Des formes avantageuses de l'invention sont décrites dans les revendications dépendantes.

L'invention est décrite çi-après à l'aide des figures qui représentent des formes non limitatives de l'invention dans lesquelles :

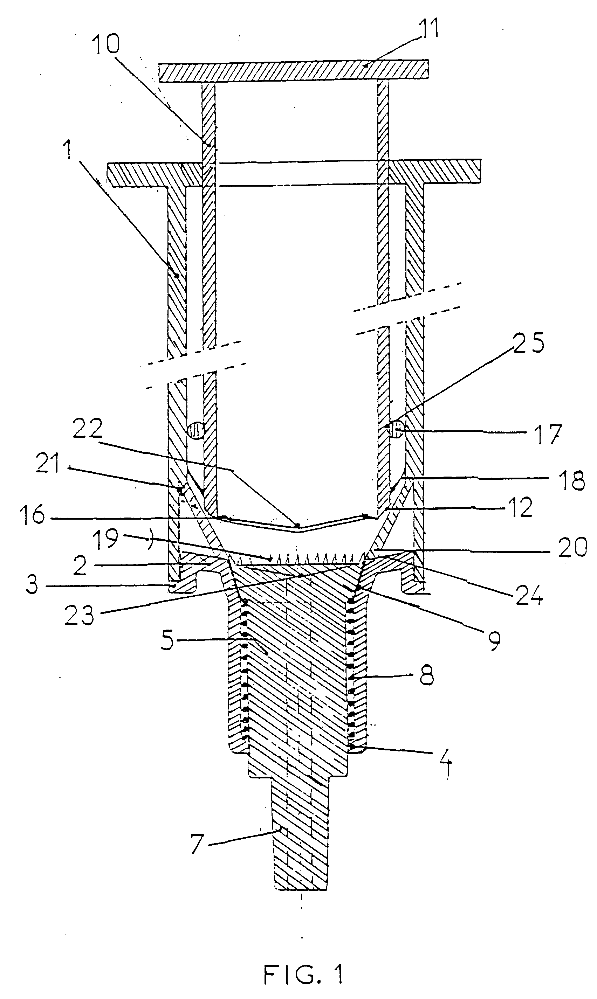

La figure 1 est une vue en coupe d'une seringue selon l' invention;

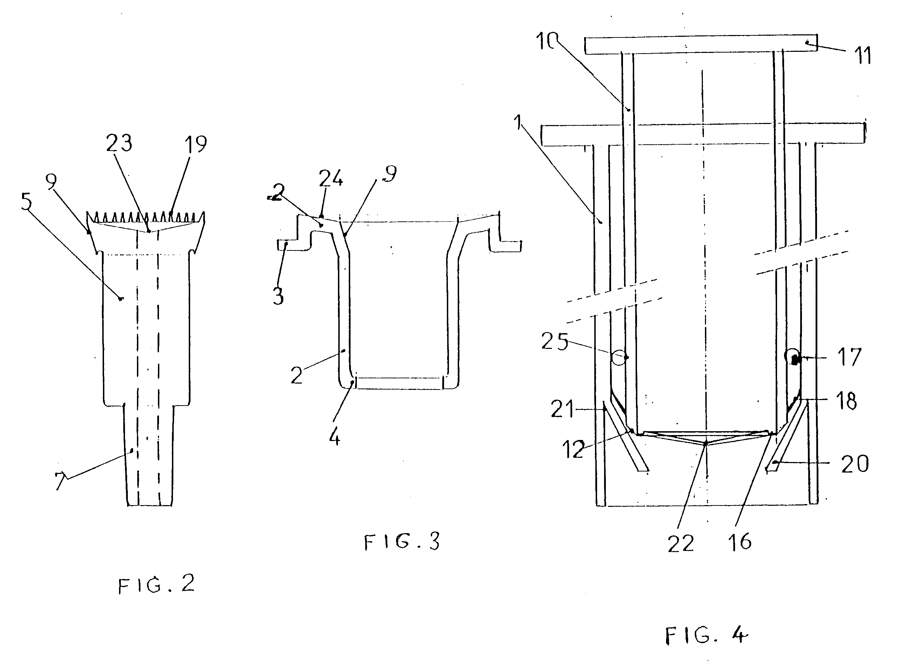

La figure 2 est une vue du porte-aiguille de la seringue Fig.1
La figure 3 est une vue de la tête de la seringue de la Fig.1
La figure 4 est une vue du corps et du piston de la seringue de la Fig.1
Le corpe de seringue (1) est constitué d'un tube cylindrique comportant deux p̂attes latèrales servant à tenir la seringue entre les doigts.

Ce corps (1) présente à son extrémité inférieure une feuillure (21) permettant son encastrement ultérieur dans la tête (2).A la naissance de la feuillure (21) plusieurs pattes (20) par exemple au nombre de trois, disposées à 120° convergent vers le centre sous un angle déterminé.

La tête (2) est formée d'une partie annulaire obtenue soit par moulage ou autre procédés. Cette tête (2) présente en extérieur une collerette de retenue (3) qui sert de butée au corps (1). L'assemblage du corps (1) avec la tête (2) peut être fait par: soudure,collage ou tout autre procédé. L'intérieur de la tête (2) présente une partie conique (24) suivie d'un deuxième cône à angle différent, constituant un plan de joint (9).

La tête se referme à son extrémité (4) pour servir de retenue à un ressort de poussée (8) et conserve une ouverture pour le passage du porte-aiguille (5). Vient ensuite le porte-aiguille (5) qui sert de raccord avec l'aiguille soit: par son embout conique (7) dénommé embout "LUER" ou, par une autre solution: le porte-aiguille (5) étant moulé sur un type d'aiguille particulière.

La tête du porte-aiguille (5) est terminé par une cône renversé (23) dont la bordure vient en prolongement de la partie cônique (24) de la tête (2). Un percement est prévu entre le sommet du cône (23) et la pointe extrème du porte-aiguille (5).

Autour du cône (23) est disposée une couronne dentée (19) ajourée de plusieurs passages pour le logement des pattes (20),correspondantes du corpe de seringue (1).

La couronne dentée (19) est destinée à la découpe du joint de fragilité (16) du piston (10).

Le piston (10) est constitué d'un tube fermé en tête par une pastille rapportée (11) qui sert de poussoir à l'utilisateur de la seringue.A son extrémité inférieure le piston (10) comporte un biseau (12) servant à la poussée oblique des pattes (20) du corps (1).

Le piston (10) est fermé à cette extrémité par une paroi (22) en forme de cône qui peut venir s'encastrer dans la partie cônique (23) du porte-aiguille (5). Cette paroi (22) comporte dans son périmètre extérieur un anneau de fragilité (16). Sur l'extérieur du piston (10) une empreinte (25) permet l'adaptation d'un joint thorique d'étancheïté (17).

En extrémité du biseau (12) du piston (10) une lèvre (18) sert à la fois de joint et de vérouillage d'anti-retour du piston (10).

A l'usage le remplissage ainsi que l'injection ne présentent aucune différence par rapport à un autre modèle courant

La purge de l'air est, elle-aussi, classique: purge qui est impossible avec les autres modèles décrits dans les brevets actuellement connus.

Lors de la fin de l'injection interviennent trois mouvements simultanés: le biseau (12) du piston vient déformer les pattes (20) qui en s'écartant vont libérer le porte-aiguille (5).

Dans le même temps la base du piston (10) présente l' anneau de fragilité (16) face à la couronne dentée (19) ce qui entraîne la rupture de l'anneau.

La partie cônique (22) est libérée en même temps que le porte-aiguille (5) qui par son ressort (8) vient se loger dans la tête du piston (10).

Cette manoeuvre entraîne l'aiguille solidaire de la partie (7) du porte-aiguille (5) rendant cet ensemble inoffensif et inaccessible.

Dans ce même temps la lèvre (18) du piston (10) vient se loger dans la feuillure (21) du corps (1). Cette lèvre (18) prenant appui sur les pattes (20) se déforme vers le centre et en inverse s'écarte entre chacune des pattes (20).

Cette manoeuvre provoque le blocage du piston (10) en interdisant son retrait.

Ce nouveau produit répond aux impératifs stricts et précis de la médecine.

## Revendications

1. Seringue anti-contagion comportant:
a) un corps (1) cylindrique assemblé à une tête (2) dans lequel est prévu une feuillure (21),
b) un porte-aiguille (5) pourvu d'un passage pour le liquide à injecter, monté couiissant dans la tête (2) maintenu dans celle-çi contre l'action d'un ressort (8),
c) un piston (10) cylindrique creux servant à la propulsion d'un liquide ou médicament, juqu'au passage du porte-aiguille (5),
d) une aiguille montée sur le porte-aiguille (5) caractérisée en ce que:
e) l'aiguille est montée amovible sur l'embout (7),
f) le porte-aiguille (5) est muni d'une partie cônique inversée dans le prolongement de la tête, en liaison avec le passage pour le liquide, pour permettre la récupération et l'évacuation de l'air résiduel,
g) le piston cylindrique comprend un fond cônique (22) en regard de la tête (2), cône comportant à sa base un anneau de fragilité (16), et, se terminant par une lèvre-joint d'étanchéïté (18) qui en fin de course du ditpiston vient se dilater dans la feuillure (21) créant ainsi un blocage anti-retour définitif,
h) le porte-aiguille (5) comporte une couronne dentée (19) en regard de l'anneau de fragilité (16), prévue pour la découpe de cet anneau (16),
i) le maintien du porte-aiguille (5) dans la tête (2) est obtenu par un dispositif de blocage conçu pour pouvoir libérer le porte-aiguille par une poussée du piston (10)
j) le piston creux (10) et la tête sont dimensionnés de façon à pouvoir loger le porte-aiguille muni de son aiguille lorsque après libération du dispositif de blocage par le piston, le porte-aiguille est propulsé sous l'effet du ressort (8) vers l'intérieur du piston (10), après avoir rompu l'anneau de fragilité (16).

2. Seringue selon la revendication 1 caractérisée en ce que le dispositif de blocage comprend des pattes (20) prenant naissance dans la feuillure circulaire (21) du corps cylindrique (1), dimensionnées de sorte que la poussée du piston (10) déplace les pattes (20) vers l'extérieur libérant ainsi le passage au porte-aiguille.

3. Seringue selon la revendication 1 caractérisée en ce que le fond (22) du piston est de forme cônique.

4. Seringue selon la revendication 1 caractérisée en ce que la tête (2) comporte une surface cônique (9) coopérant avec une surface cônique du porte-aiguille de manière à constituer un joint d'étanchéïté.

5. Seringue selon la revendication 2 caractérisée en ce que le piston (10) comporte une lèvre (18) qui subit une double déformation par appui sur les pattes (20) et se bloque dans la feuillure (21).

## Claims

1. The anti-infectious syringe includes:
a) a cylindrical body (1) assembled to a head (2) in which has been forecasted a groove (21),
b) a needle-holder (5) with some outlet for the liquid to be injected, mounted sliding in the head (2), maintained within it against the action of a spring (8),
c) a hollow piston valve (10) that is used for the propelling of a liquid or a medicine till the passage of the needle-holder (5),
d) a needle mounted on the needle-holder (5) that is characterized by :
e) the needle which is mounted and to be removable on the tip (7),
f) the needle-holder (5) which is provided with a conical part inverted in the continuation of the head, in connection with the outlet for liquid, so as to allow the recovery and the emptying of the residual air,
g) the piston valve is composed of a conical base (22) facing the head (2), cone composed of a fragility ring (16) and that ends by a lipped air seal (18) which, at the end of the stroke of the piston, will expand in the groove (21) so creating a definite anti-return blocking,
h) the needle-holder (5) is composed of a rim collar facing the fragility ring (16), aimed at the cutting of this ring (16),
i) the holding of the needle-holder (5) in the head (2) is obtained thanks to a blocking device conceived to make it possible to release the needle-holder by a thrust of the piston (10)
j) the hollow piston (10) and the head are sized in order to be able to part the needle-holder with its spike when, after release of the blocking device by the piston, the needle-holder is thrusted under the effect of the spring (8) towards the interior of the piston (10), after having broken the fragility ring (16).

2. The syringe, according to the demand n° 1 is characterized by the fact that the blocking device is composed of clutches (20) deriving from the circular groove (21)of the cylindrical body (1), sized so that the thrust of the piston (10) shifts the clutches (20) towards the exterior releasing that way the outlet of the needle-holder.

3. The syringe, according to the demand n° 1 is characterized by the conical shape of the piston's base (22).

4. The syringe, according to the demand n° 1 is characterized by the head (2) which is composed of a conical surface (9) cooperating with a conical surface of the needle-holder so as to constitute an air seal.

5. The syringe, according to the demand n° 2, is characterized by the piston (10) composed of a lip (18) which undergoes a double strain by resting on the clutches (20) and which jams the groove(22).

## Patentansprüche

1. Anti-Kontaminierungsspritze bestehend aus :
a) einem zylindrischen Körper (1) mit einem angefügten Kopfteil (2) worin sich eine ringförmige Nut (21) befindet.
b) einem Nadelsockel (5) in dem sich ein Durchfluss für die Injektionsflüssigkeit befindet der in den Kopfteil (2) gegen den Druck einer Feder (8) eingepfropft ist.
c) einem hohlen zylindrischen Kolben (10) zur Förderung der Injektionsflüssigkeit bis zum Erreichen des Nadelsockels (5)
d) einer Nadel,die auf den Nadelsockel (5) aufgesteckt ist.
e) diese Nadel ist mit dem Nadelsockel (5) nur durch Aufstecken verbunden.
f) der Nadelsockel (5) weist axial zum Kopfteil (2) ausgerichtet, eine konische Höhlung auf, um in Verbindung mit dem Durchfluss für die Injektionsflüssihkeit Gas-und Flüssigkeitsreste aufsunehmen.
g) der zylindrische Kolben weist ein konisches Kopfstücks (22) auf,unterhalb dessen sich die Sollbruchstelle des Pleuels (16) befindet. Um den Rand des Kopfstücks (22) ist ein elastischer Drichtring (18) angebracht, der sich, bei völliger Entleerung der Spritze,in die Nut (21) ausdehnt und so den Holben endgültig im Zylinder blockiert.
h) der Nadelsockel (5) weist gegenüber der Sollbruchstelle (16) eine gezahnte Krone (19) auf, die die Herbeiführung des Sollbruchs (16) ermöglicht.
i) die Befestigung des Nadelsockels (5) im Kopfteil (2) erfolgt mittels Widerhaken dergestalt,dass ein wollständiger Kolbenhub (10) den Nadelsockel (5) freigibt.
j) der hohle zylindrische Kolben (10) ost so gross dass er den Nadelsockel (5) samt Nadel in sich aufnehmen kann,wenn bei vollständigem Kolbenhub (10) nach Freigabe des Nadelsockels (5) mittels des Zahnkranzes (19) der Sollbruch (16) erfolgt, und der Nadelsockel (5) durch die Federspannung (8) ins Innere des Kolben (10) gestrieben wird.

2. Injektionsspritze gemass der Inanspruchnahme 1 insofern die Befestigungsvorrichtung Widerhaken (20) aufweist,die in die Nut (21) des zylindrischen Körpers (1) greifen,und die so geartet sind, dass der Kolbenhub (10) die Widerhaken (20) nach aussen hin freisetzt und so die Befestigung des Nadelsockels aufhebt.

3. Injektionsspritze gemäss der Inanspruchnahme 1 insofern zynlindrischen Kolben ein konisches Kopfstück (22) aufweist.

4. Injektionsspritze gemäss der Inanspruchnahme 1 insofern das Kopfteil (2) eine konische Oberfläche aufweist, die zusammen mit einer konischen Fläche des Nadelsockels (5) eine Dichtvorrichtung ergibt.

5. Injektionsspritze gemäss der Inanspruchnahme 2 insofern der Kolben (10) einen elastischen Dichtring (18) aufweist, der durch seine Anlagerung an die Widerhaken (20) eine doppelte Verformung erfährt und sich so endgültig in der Nut (21) festsetz.
